# DEMANDE DE BREVET EUROPEEN

(11) **EP 1 586 271 A1**
(43) Date de publication de la demande: **19.10.2005**
(21) Numéro de dépôt: 05290829.0
(22) Date de dépôt: 14.04.2005
(51) Int. Cl.: A61B 17/00

(54) **Dispositif d'eveinage**

(30) Priorité: 16.04.2004 FR 0404025
(71) Demandeur: M2CT (Sarl), 59175 Templemars (FR)
(72) Inventeur: Camus, Michel, 59175 Templemars (FR); Mikati, Assem, 59177 Sains du Nord (FR)
(74) Mandataire: Blot, Philippe Robert Emile

(57) **Abrégé**

II s'agit d'un éveineur, stripper, utilisant une bande absorbante et/ou coagulante pour diminuer les risques d'hématomes. Ledit éveineur comprend un fil ou câble d'extraction de veine relié à son extrémité à une bande de matériau naturel ou synthétique, matériau agrippant, absorbant le sang, de longueur nécessaire pour l'éveinage, supportant et agrippant la veine pour son invagination progressive sur tout ou partie de sa longueur, lors de la progression de l'éveinage.

## Description

La dégradation des veines saphènes des jambes et les douleurs qu'elles entraînent ont amené le corps médical à pratiquer un éveinage total ou partiel des veines saphènes ceci afin de supprimer soit la compression du nerf saphène qui jouxte les veines, source de douleur, soit l'aspect disgracieux des varices, ampoules ressemblant à une hernie, d'un ou de plusieurs endroits de la veine concernée.

Cette opération appelée Eveinage ou Stripping, pratiquée le plus souvent par des chirurgiens, se fait depuis très longtemps et le matériel qui a permis de réaliser cet acte chirurgical a évolué dans le temps.

Les premiers strippers étaient composés d'une olive de plus ou moins grande taille que l'on fixait sur un câble et que l'on introduisait dans la jambe, au niveau supérieur ou inférieur de la veine concernée.

Une traction sur le câble entraînait la boule ainsi fixée et "arrachait littéralement" la veine de son emplacement.

Cette méthode d'intervention toujours pratiquée hélas, car de bas prix, est cependant en voie de régression, au bénéfice du stripping par invagination.

Cette méthode consiste à replier la veine à l'intérieur d'elle-même donc à occasionner beaucoup moins de traumatismes et de saignements et une nette diminution des risques de paresthésie ou d'excès de sensibilisation terminale du pied que la méthode initiale.

Cependant une veine pathologique, fragile, ou une disposition de la veine à se rompre lors de la traction, nécessite alors, dans le même temps opératoire, l'utilisation d'une des olives qui accompagnent ce type de stripper, et contraint donc le praticien à terminer le stripping de la façon précitée. (cf Prof Chevalier, Prof Becquemin, Dr Valici etc...)

Les risques encourus sont moindres car une partie de la veine a déjà été extraite par cette méthode, ce n'est donc que le reliquat veineux toujours en place, donc moins volumineux, qu'il faut seulement retirer.

Nous proposons donc une autre alternative à ce type de stripping, tout en en conservant le principe de l'invagination.

L'invention sera mieux comprise à la lecture de la description qui va suivre donnée à titre d'exemple et faite en se référant aux dessins sur lesquels :
- la Figure 1.1 est une vue partielle en élévation d'un instrument chirurgical d'éveinage selon l'invention ;
- la Figure 1.2 est une vue identique à celle de la Figure 1 d'une variante de réalisation d'un instrument chirurgical d'éveinage ;
- les Figures 2.1, 2.2, 2.3, 2.4 et 2.5 sont des vues en élévation d'autres variantes de réalisation ; et
- les Figures 3.1 et 3.2 sont encore d'autres variantes de réalisation.

C'est ainsi que l'instrument chirurgical d'éveinage ou stripper pourrait se composer d'un câble 12, ou fil de traction, matériel qui sert toujours dans les matériels précités.

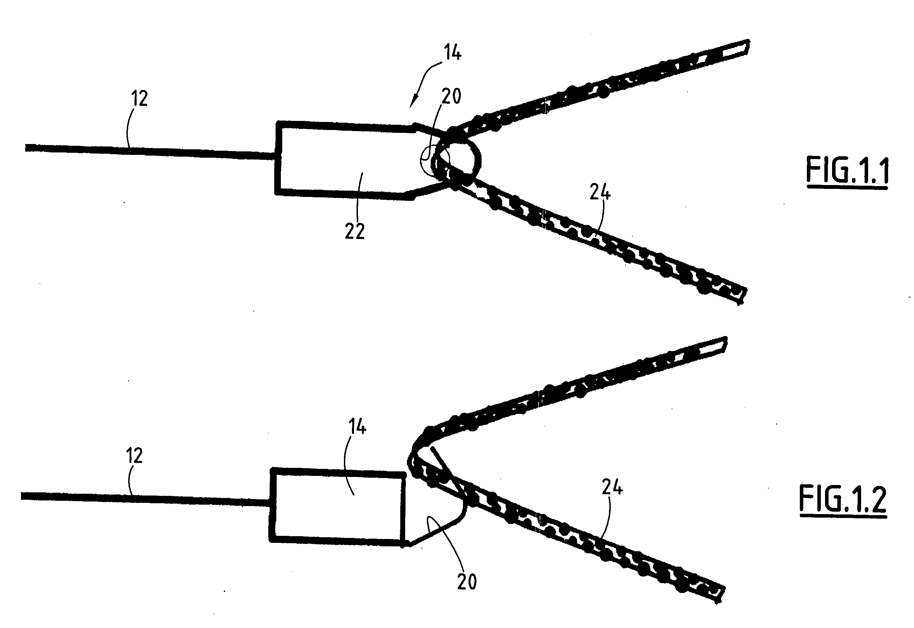

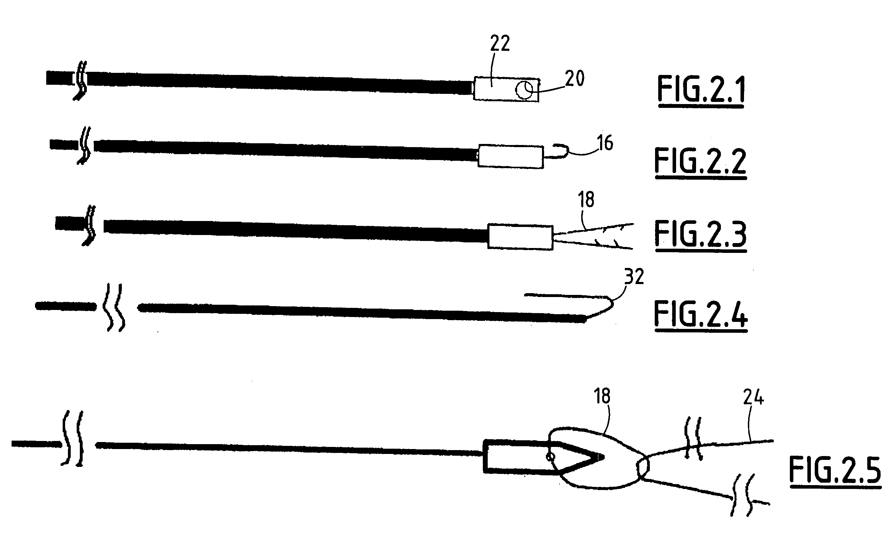

Celui ci serait pourvu en sa terminaison d'un dispositif 14 tel un crochet 16 (Figure 2.2) ou une pince de préhension (Figures 2.3) ou un anneau pivotant 18 (Figure 2.5) ou tout autre dispositif tel un orifice suffisant 20, dans un embout 22 (Figures 1.1 et 2.1) pouvant permettre de fixer, ou enfiler dans sa prolongation une bande 24 de crêpe ou une bande de gaze, suffisamment longue pour dépasser largement la longueur du membre concerné et plus précisément la longueur de la veine à extraire. La longueur de la bande 24 s'entend de la bande repliée, c'est-à-dire la distance séparant la partie repliée de la bande de son extrémité libre la plus éloignée si les deux brins ont des longueurs différentes. En pratique, les deux brins ont avantageusement une même longueur propre à égale à la longueur de la bande.

La bande est avantageusement en crêpe plutôt qu'en gaze, le crêpe étant un tissu présentant un aspect ondulé caractéristique, obtenu par l'emploi de fils à forte torsion, dits "fils crêpe".

Le stripping se ferait alors sur la bande et non plus sur un fil ou sur un tube.

La qualité de cette bande et l'épaisseur qu'elle représentera sera nécessaire et suffisante pour que la veine fasse corps avec celle-ci et soit extraite par retournement comme décrit précédemment.

La bande en question serait avantageusement de longueur nécessaire et suffisante pour que, la totalité de la veine étant sortie, le reste de la bande soit toujours en place dans la jambe et y resterait un certain temps, le temps nécessaire à la phlebectomie associée par exemple, et sa capillarité serait mise à contribution pour absorber le sang, directement in situ, et diminuer en plus les risques d'hématomes post-opératoires toujours présents. La bande sera donc de nature absorbante.

L'instrument chirurgical d'éveinage comprend un fil ou câble d'extraction de veine relié à son extrémité à une bande 24 de matériau naturel ou synthétique, matériau agrippant, (Figure 1.3) absorbant le sang, de longueur nécessaire pour l'éveinage, supportant et agrippant la veine au fur et à mesure de son invagination progressive sur tout ou partie de sa longueur, lors de la progression de l'éveinage. On peut même concevoir une bande imprégnée d'un agent de coagulation qui faciliterait donc, pendant que l'élément de la bande restant , reste in situ, faciliterait donc la coagulation des tissus environnants et des sites de rupture des collatérales restées en place, source de saignement per et post-opératoires parfois importants.

La longueur de la bande de l'instrument serait supérieure à la longueur de la veine, pour, une fois la veine extraite, permettre à la partie de la bande située et restant dans la zone concernée, d'absorber le sang. Avantageusement la longueur de la bande est au moins deux fois supérieure à la longueur de la veine à extraire.

Tout ou partie de ladite bande de l'instrument comprend au moins un agent favorisant la coagulation du sang in situ

L'instrument comprend un dispositif de liaison entre le fil et la bande, tel un anneau pivotant sur le fil, ou un orifice 20 ouvrable (Figure 1.2) ou non (Figures 1.1 et 2.1), pratiqué dans l'embout, la bande s'insérant dans l'anneau ou l'orifice par pliage

La bande de matériau absorbant de l'instrument est un matériau textile non lisse et de type "crêpe" car ses aspérités favorisent alors l'adhésivité de la veine sur ladite bande.

D'autre-part, la nécessité d'avoir en "roue de secours" une olive pour le cas ou la veine casserait ne s'avère plus nécessaire car la veine, sur tout le trajet de la bande, et par sa distension, ne demandera qu'à adhérer au tissus agrippant donc à diminuer la force de traction initiale représentée par la seule ligature de la veine sur le fil de traction. D'autre part, un simple noeud réalisé sur la partie inférieure de la bande par exemple, fera le même office tout en étant moins traumatisant car moins rigide. Il suffira alors au praticien de retirer cette bande restée en place, en fin d'intervention pour obtenir un lit jambier sans excédent de sang donc sans hématome, risque médical et toujours disgracieux.

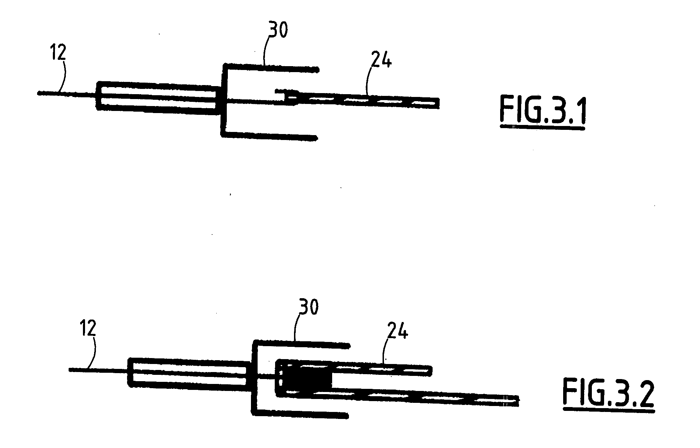

Le dispositif de solidarisation du fil ou câble et de la bande est recouvert d'un élément 30 (Figures 3.1, 3.2) propre à faciliter l'introduction du dispositif dans la veine concernée ; le fil ou câble utilisé noté 32 sur la Figure 2.4 est déformable avec une pince pour former une butée pour le tube coulissant qui l'entourerait.

On comprend, qu'avec un tel instrument, la veine retournée s'appuie intérieurement sur la bande, laquelle par sa structure épouse la forme de la veine et renforce celle-ci évitant les ruptures accidentelles.

## Revendications

1. Instrument chirurgical d'éveinage, **caractérisé en ce qu'**il comprend un fil ou câble (12 ; 32) d'extraction de veine relié à son extrémité à une bande (24) de matériau naturel ou synthétique, matériau agrippant, absorbant le sang, de longueur nécessaire pour l'éveinage, supportant et agrippant la veine pour son invagination progressive sur tout ou partie de sa longueur, lors de la progression de l'éveinage.

2. Instrument selon la revendication 1, **caractérisé en ce que** la longueur de la bande (24) est supérieure à la longueur de la veine, pour, une fois la veine extraite, permettre à la partie de la bande située et restant dans la zone concernée, d'absorber le sang.

3. Instrument selon la revendication 1 ou 2, **caractérisé en ce que** tout ou partie de ladite bande (24) comprend au moins un agent favorisant la coagulation du sang in situ.

4. Instrument selon l'une quelconque des revendications précédentes, **caractérisé en ce qu'**il comprend un dispositif (14) de liaison entre le fil (12) et la bande (24).

5. Instrument selon la revendication 4, **caractérisé en ce que** le dispositif de liaison (14) entre le fil (12) et la bande (24) est un anneau pivotant (18 ; 20) sur le fil, la bande (24) s'insérant dans l'anneau (18, 20) par pliage.

6. Instrument selon l'une quelconque des revendications précédentes, **caractérisé en ce que** la bande (24) de matériau absorbant est un matériau textile non lisse et absorbant de type "crêpe".

7. Instrument selon la revendication 4, **caractérisé en ce qu'**il comprend un crochet (16) de solidarisation du fil (12) de traction et de la bande (24).

8. Instrument chirurgical d'éveinage selon la revendication 4, **caractérisé en ce que** le dispositif de solidarisation du fil de traction et de la bande est une pince (18).

9. Instrument chirurgical d'éveinage, selon l'une quelconque des revendication 4, 5, 7 ou 8, **caractérisé en ce que** le dispositif de solidarisation du fil ou câble, et, de la bande, est recouvert d'un élément (30) propre à faciliter l'introduction du dispositif dans la veine concernée.

10. Instrument chirurgical d'éveinage selon la revendication 1, **caractérisé en ce que** le fil ou câble utilisé (32) est déformable avec une pince pour former une butée pour le tube coulissant qui l'entourerait.
